# EUROPEAN PATENT APPLICATION

(11) **EP 2 601 913 A1**
(43) Date of publication of application: **12.06.2013**
(21) Application number: 11814516.8
(22) Date of filing: 27.07.2011
(51) Int. Cl.: A61F 5/01, A61H 39/04

(54) **POSTURE-CORRECTING TOOL**

(30) Priority: 03.08.2010 JP 2010174482
(71) Applicant: Tominaga, Hirofumi, Tokyo 106-0032 (JP)
(72) Inventor: Tominaga, Hirofumi, Tokyo 106-0032 (JP)
(74) Representative: Grünecker, Kinkeldey, Stockmair & Schwanhäusser
(86) International application number: PCT/JP2011/067083
(87) International publication number: WO 2012/017889

(57) **Abstract**

The present invention provides a posture correcting tool which has a simple structure and can be used easily. The posture correcting tool includes an attachment member 10 attachable to the head of a user and at least one pressing portion 20 which is provided in the attachment member 10, protrudes from the attachment member 10 toward the head of the user, and can press at least a portion of a peripheral surface of the head including the forehead of the user. The attachment member 10 is formed of an elastic material formed into a ring shape attachable to the peripheral surface of the head of the user, and the pressing portion 20 presses the head of the user with contractive force of the attachment member 10.

## Description

### Technical Field

The present invention relates to a posture correcting tool which is used by being attached to the head of a user and corrects the posture of the user.

### Background Art

In recent years, a health hazard due to poor posture has been seen as a problem, and there has been known that particularly a so-called hunchback leads to various harmful effects such as shoulder stiffness, backache, bowleg, and an internal disease and significantly impacts on human health.

Conventional treatments of symptoms such as shoulder stiffness include stimulation of pressure points of the human body by acupressure and massage for reducing rigid muscle tension. However, the treatments including massage are just temporary responses, and the symptoms such as shoulder stiffness will recur unless a deteriorated posture is corrected.

Meanwhile, various posture correcting tools for correcting the posture of a user have been used (see, Patent Literatures 1 and 2). The posture correcting tools disclosed in the Patent Literatures 1 and 2 are used by being attached to a trunk of the user, and the shoulders of the user are raised backward by utilizing contractive force of a belt wrapped around the shoulders of the user, whereby curvature of the back muscles is corrected.

### Citation List

### Patent Literature

Patent Literature 1: Japanese Patent Application Laid-Open No. 9-75383
Patent Literature 2: Japanese Examined Utility Model Application Publication No. 5-29705

### Summary of Invention

### Technical Problem

However, the posture correcting tools disclosed in the Patent Literatures 1 and 2 have a complex structure, and, at the same time, it is difficult to wear them. Moreover, there is a problem that once a user wears the posture correcting tools, the user cannot easily remove the posture correcting tools. Although in the posture correcting tools disclosed in the Patent Literatures 1 and 2 the shoulders of a user can be raised by the contractive force of the belt, there is a problem that forward tilting of the head, distortion, and the like as symptoms of a so-called hunchback cannot be corrected.

Thus, an object of the present invention is to provide a posture correcting tool which has a simple structure and can be used easily.

### Solution to Problem

In order to achieve the above object, a posture correcting tool according to the present invention is characterized by including: an attachment member attachable to a head of a user; and at least one pressing portion which is provided in the attachment member, protrudes from the attachment member toward the head of the user, and can press at least a portion of a peripheral surface of the head including a forehead of the user. Here, the peripheral surface of the head including the forehead of the user means the forehead of the user and regions of a temporal region and an occipital region which are located at a height comparable to the height of the forehead.

As described above, in the posture correcting tool according to the present invention, an attachment member is attached to the head of a user, whereby a pressing portion presses the peripheral surface of the head including the forehead of the user. Therefore, the head of the user can be guided to an ideal position, and the posture of the user can be corrected. Further, since in the posture correcting tool according to the present invention the peripheral surface of the head including the forehead of the user is pressed to correct the posture of the user, the structure is simple. Furthermore, the posture correcting tool can be used by such a simple operation as attaching the posture correcting tool to the head of the user.

In the posture correcting tool according to the present invention, it is considered that the posture of the user according to a so-called "hanger reflex" or a phenomenon similar to the hanger reflex can be corrected. In the "hanger reflex", when at least a portion of the head of a human being is compressed, the brain is stimulated, and the head unconsciously swivels in any of the up, down, left and right directions.

In the posture correcting tool according to the present invention, at least two pressing portions may be provided along the peripheral surface of the head including the forehead of the user at a distance in the circumferential direction. Further, in the posture correcting tool according to the present invention, at least two pressing portions may be provided along the peripheral surface of the head including the forehead of the user at a distance in the circumferential direction, in this case, at least two pressing portions may be provided at a distance in a direction that is perpendicular to the circumferential direction and parallel to the peripheral surface of the head including the forehead of the user.

In the posture correcting tool according to the present invention, it is preferable that the pressing portion is configured so that an interval between the pressing portion and another adjacent pressing portion can be adjusted. The pressing portion is thus configured so that the interval between the pressing portion and another adjacent pressing portion can be adjusted, whereby since the pressing portion can be adjusted to a position where the effect of the posture correction is best exercised, the effect of the posture correction can be further exercised.

Further, in the posture correcting tool according to the present invention, it is preferable that the pressing portion is configured so that the position with respect to the peripheral surface of the head including the forehead of a user can be adjusted. The pressing portion is configured so that the position with respect to the peripheral surface of the head including the forehead of a user can be adjusted, whereby since the pressing portion can be adjusted to the position where the effect of the posture correction is best exercised, the effect of the posture correction can be further exercised.

Further, in the posture correcting tool according to the present invention, the attachment member may be formed into a ring shape attachable to the peripheral surface of the head including the forehead of a user or may be formed into a hat shape attachable to the head of the user. The attachment member is formed into a ring shape attachable to the peripheral surface of the head including the forehead of the user, whereby the posture correction tool having a simple structure can be provided. Meanwhile, when the attachment member is formed into a hat shape attachable to the head of a user, the posture correcting tool having excellent appearance can be provided, and the posture correcting tool can be actively used in form correction in sports and the like.

Furthermore, in the posture correcting tool according to the present invention, it is preferable that at least a portion of the attachment member is formed of an elastic material, and the pressing portion is configured to press at least a portion of the peripheral surface of the head including the forehead of a user by the contractive force of the attachment member. The pressing portion presses the peripheral surface of the head including the forehead of a user with the contractive force of the attachment member formed of the elastic material, an actuator for pressing the pressing portion is not required, and therefore, a posture correcting tool having a simpler structure can be provided.

### Advantageous Effects of Invention

As described above, the present invention can provide a posture correcting tool which has a simple structure and can be used easily.

### Brief Description of Drawings

FIG. 1 is a perspective view showing a posture correcting tool according to a first embodiment of the present invention.
FIG. 2 is a perspective view showing a state in which the posture correcting tool according to the first embodiment is attached to the head of a user.
FIG. 3 is a side view showing a process for correcting a posture of the user with the use of the posture correcting tool according to the first embodiment.
FIG. 4 is a perspective view showing another example of the posture correcting tool according to the first embodiment.
FIG. 5 is a perspective view showing a posture correcting tool according to a second embodiment of the present invention.
FIG. 6 is a perspective view showing a posture correcting tool according to a third embodiment of the present invention.
FIG. 7 is a perspective view showing a posture correcting tool according to a fourth embodiment of the present invention.

### Description of Embodiments

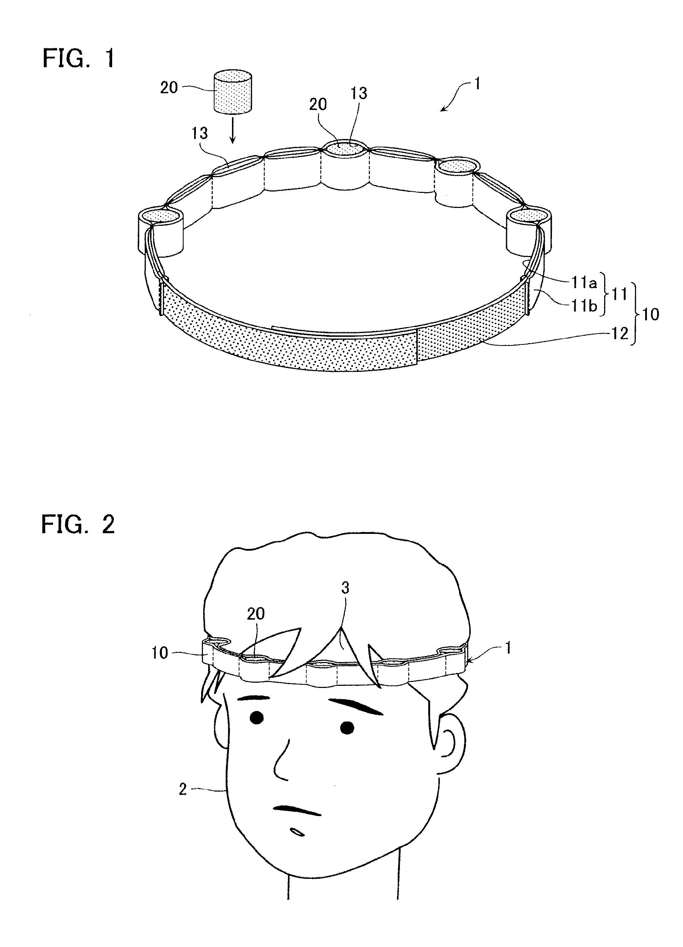

Next, a posture correcting tool according to a first embodiment of the present invention will be described based on the drawings. As shown in FIGS. 1 and 2, a posture correcting tool 1 according to the first embodiment includes an attachment member 10 attachable to the head of a user 2 and one or a plurality of pressing portions 20 protruded from the attachment member 10 toward the head of the user 2.

The attachment member 10 is constituted of a belt-like attachment member body 11 formed of an elastic material such as woven rubber and hook-and-loop fasteners 12 provided at both the ends of the attachment member body 11 and is formed into a ring shape attachable to the peripheral surface of the head including a forehead 3 of the user 2. The attachment member body 11 has a double structure including an inner band 11a and an outer band 11b which are formed into a belt shape, and the attachment member body 11 has such a contractility that suitably biases the pressing portion 20 to the peripheral surface of the head including the forehead 3 of the user 2 when the attachment member body 11 is attached to the head of the user 2. The peripheral surface of the head including the forehead 3 of the user 2 means the forehead 3 of the user 2 and regions of a temporal region and an occipital region which are located at a height comparable to the height of the forehead. In the hook-and-loop fastener 12, a hook-and-loop fastener provided at one end of the attachment member body 11 and a hook-and-loop fastener provided at the other end are engaged with each other, whereby the attachment member 10 is converted into a ring shape, and, at the same time, the diameter of the attachment member 10 can be adjusted.

The attachment member body 11 is sewn so that the inner band 11a and the outer band 11b are equally divided into eleven portions in the longitudinal direction, whereby eleven holding portions 13 are formed. In each of the holding portions 13, the pressing portion 20 is held between the inner band 11a and the outer band 11b in an insertable and removable manner by the contractive force of the inner band 11a and the outer band 11b.

The pressing portion 20 is formed of resin or the like and formed into a columnar shape. The peripheral surface of the pressing portion 20 is held by each of the holding portions 13, whereby when the attachment member 10 is attached to the head of the user 2, the pressing portion 20 is biased by the attachment member 10, and the peripheral surface of the head including the forehead 3 of the user 2 can be pressed.

The posture correcting tool 1 according to the first embodiment is attached to the head including the forehead 3 of the user 2 in such a state that one or a plurality of the pressing portions 20 are held by one or a plurality of the holding portions 13, whereby at least a portion of the peripheral surface of the head including the forehead 3 of the user 2 is pressed by the pressing portion 20. In the posture correcting tool 1 according to the first embodiment, the holding portion 13 holding the pressing portion 20 is suitably selected, whereby the position of the pressing portion 20 with respect to the peripheral surface of the head including the forehead 3 of the user 2 and an interval between the pressing portions 20 adjacent to each other can be adjusted.

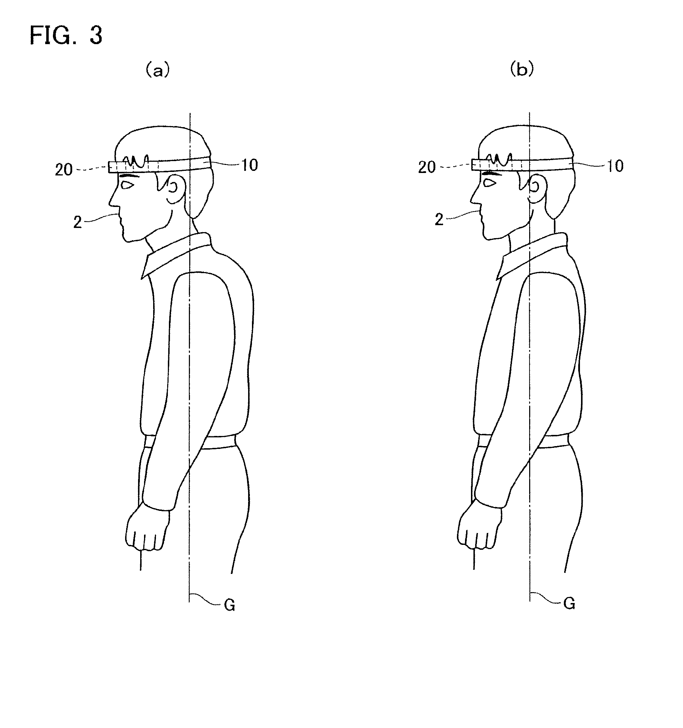

Next, the usage of the posture correcting tool 1 according to the first embodiment will be described using FIG. 3. As shown in FIG. 3 (a), for example, the posture correcting tool 1 according to the first embodiment is used by the user 2 whose head position is deviated from the center of gravity G of the body by forward tilting of the head and distortion. Specifically, the pressing portion 20 is first held by the desired holding portion 13 of the attachment member 10. At this time, the number of the pressing portions 20 and the position of the holding portion 13 can be selected according to the degree of the symptom of the user 2, a portion that the user wants to treat, and the like. Then, the attachment member 10 is converted into a ring shape by the hook-and-loop fastener 12 and attached to the peripheral surface of the head including the forehead 3 of the user 2. Then, the attachment member 10 is adjusted by being moved along the peripheral surface of the head including the forehead 3 of the user 2, and each of the pressing portions 20 is pressed against the forehead 3 of the user 2 at a desired position. According to this constitution, as shown in FIG. 3 (b), the head of the user 2 is unconsciously swiveled upward, and the posture of the user 2 can be corrected into a suitable posture, that is, a posture matching the position of the head to the center of gravity G of the body.

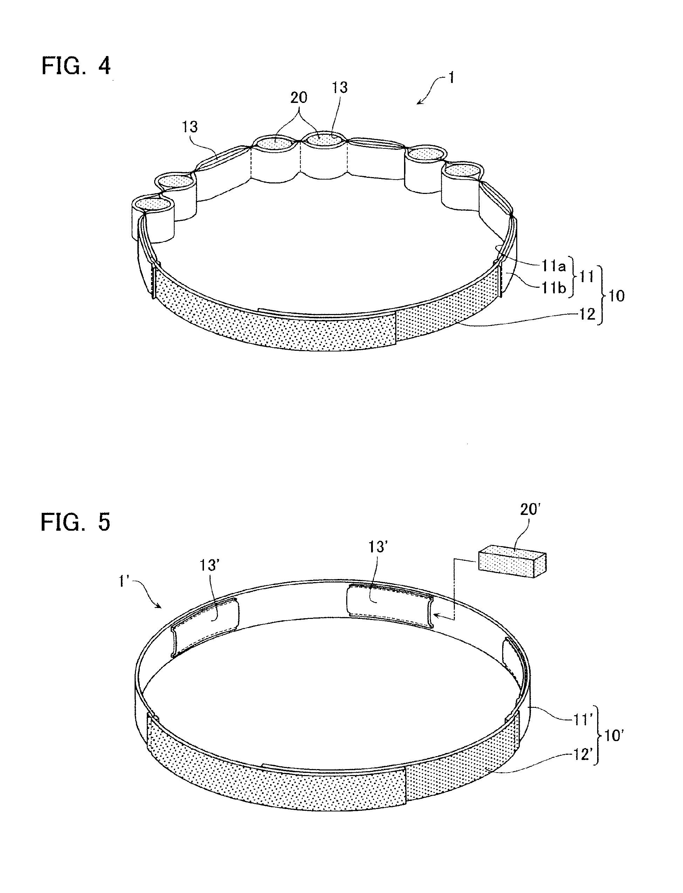

In the posture correcting tool 1 according to the first embodiment, as shown in FIG. 4, for example, the attachment member body 11 is formed of an elastic member having a relatively high contractive force. At the same time, every two of the pressing portions 20 are arranged to be adjacent to each other, and the pressing area and the pressing force at one portion are increased, whereby a high effect on users with a large muscle mass such as athletes can be exercised.

Next, a posture correcting tool according to a second embodiment of the present invention will be described based on FIG. 5. In a posture correcting tool 1' according to the second embodiment, the detail description of the configuration similar to that of the posture correcting tool 1 according to the first embodiment will be omitted, and only different configurations will be described. As shown in FIG. 5, the posture correcting tool 1' according to the second embodiment includes an attachment member 10' attachable to the head of a user 2 and a pressing portion 20' protruded from the attachment member 10' toward the head of a user. The attachment member 10' is constituted of a belt-like attachment member body 11' formed of an elastic material such as woven rubber and hook-and-loop fasteners 12' provided at both the ends of the attachment member body 11' and is formed into a ring shape attachable to the peripheral surface of the head including the forehead of the user.

The attachment member body 11' is different from the attachment member body 11 of the posture correcting tool 1 according to the first embodiment, is constituted of a single band formed into a belt shape (that is, a band which does not have a double structure), and has such a contractility that suitably biases the pressing portion 20' to the peripheral surface of the head including the forehead of the user when the attachment member body 11' is attached to the head of the user. The inner peripheral surface of the attachment member body 11' includes at least one holding portion 13' holding the pressing portion 20' in an insertable and removable manner, and in the present embodiment the three holding portions are provided at regular intervals in the circumferential direction. Regarding the holding portion 13' , an upper side and a lower side of a rectangular woven rubber piece elongated in the circumferential direction is sewn on the inside of the attachment member body 11' to form the holding portion 13' into a pouch shape in and from which the pressing portion 20' can be inserted and removed in the circumferential direction. In the attachment member 10', the pressing portion 20' can be held between the attachment member body 11' and the holding portion 13' by the contractive force of the attachment member body 11' and the holding portion 13'.

The pressing portion 20' is formed of resin or the like and formed into a prismatic shape elongated in the circumferential direction. The pressing portion 20' is formed to have a size storable in the holding portion 13' and a length smaller than the length in the circumferential direction of the holding portion 13'. The size of the pressing portion 20' can be suitably changed according to the application and usages.

The posture correcting tool 1' according to the second embodiment is attached to the head including the forehead of a user in such a state that one or a plurality of the pressing portions 20' are held by one or a plurality of the holding portions 13', whereby at least a portion of the peripheral surface of the head including the forehead of the user is pressed by the pressing portion 20'. In the posture correcting tool 1' according to the second embodiment, the pressing portion 20' is formed into a prismatic shape elongated in the circumferential direction, and the pressing area of the pressing portion 20' is increased, whereby a high effect on users with a large muscle mass such as athletes can be exercised. In the posture correcting tool 1' according to the second embodiment, the holding portion 13' holding the pressing portion 20' is suitably selected, whereby the position of the pressing portion 20' with respect to the peripheral surface of the head including the forehead of a user and an interval between the pressing portions 20' adjacent to each other can be adjusted.

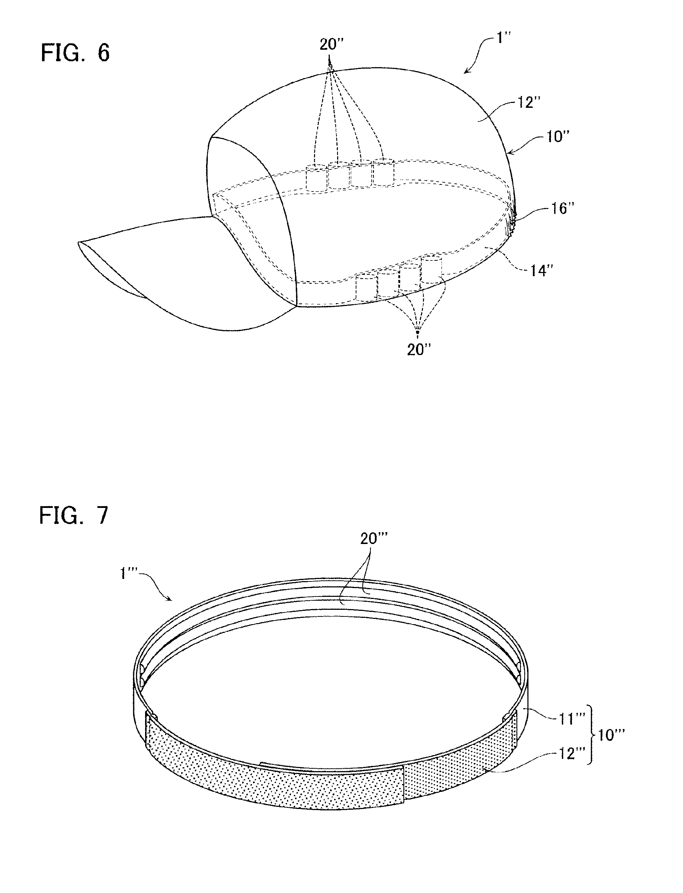

Next, a posture correcting tool according to a third embodiment of the present invention will be described based on FIG. 6. An attachment member 10" of a posture correcting tool 1'' according to the third embodiment is different from the first embodiment and is formed into a hat shape attachable to the head of a user. A belt-like inner band 14" is provided inside a peripheral edge of a hat body 12'' of the attachment member 10" throughout the entire circumference in the circumferential direction. The inner band 14" has predetermined contractive force, and the pressing portion 20" is held in an insertable and removable manner between the inner band 14" and the inside of the peripheral edge of the hat body 12''. On the backward of the peripheral edge of the hat body 12" (rightward in FIG. 6), a stretching portion 16" stretchable in the circumferential direction is provided by attaching a rubber member or the like. The stretching portion 16" has such a contractility that suitably biases the pressing portion 20'' to the peripheral surface of the head including the forehead of a user when the hat body 12" is attached to the head of the user. Although the stretching portion 16'' is provided on the backward of the peripheral edge of the hat body 12", the present invention is not limited thereto, and the stretching portion 16" can be provided in any portion, or the stretching portion 16'' may not be provided. In the posture correcting tool 1'' according to the third embodiment, since the pressing portion 20" is held between the inside of a peripheral edge of the hat body 12'' and the inner band 14", restrictions of the shape of the pressing portion 20" can be reduced, and the pressing portions 20" having various shapes can be adopted.

The posture correcting tool 1'' according to the third embodiment is attached to the head including the forehead of a user in such a state that one or a plurality of the pressing portions 20'' are held between the inside of the peripheral edge of the hook-and-loop fastener 12" and the inner band 14", whereby at least a portion of the peripheral surface of the head including the forehead of the user is pressed by the pressing portion 20''. According to the posture correcting tool 1'' according to the third embodiment, since the pressing portion 20'' is held between the inside of the peripheral edge of the hat body 2'' and the inner band 14'', each of the pressing portions 20" is moved in the circumferential direction between the inside of the peripheral edge of the hat body 12'' and the inner band 14'', whereby the position and the interval of the pressing portion 20" can be easily adjusted. In the posture correcting tool 1'' according to the third embodiment, since the pressing portion 20" is inserted and removed freely, the number of the pressing portions 20" can be suitably adjusted depending on the situation.

Next, a posture correcting tool according to a fourth embodiment of the present invention will be described based on FIG. 7. In a posture correcting tool 1''' according to the fourth embodiment, the detail descriptions of the configurations similar to those of the posture correcting tool 1, 1', and 1'' of the first to third embodiments will be omitted, and only different configurations will be described. As in the posture correcting tool 1' according to the second embodiment, an attachment member 10''' of the posture correcting tool 1''' according to the fourth embodiment is constituted of an attachment member body 11''' formed into a belt shape and formed of a single band and hook-and-loop fasteners 12''' provided at both the ends of the attachment member body 11''' and is formed into a ring shape attachable to the peripheral surface of the head including the forehead of a user. The inner peripheral surface of the attachment member body 11''' includes at least one pressing portion 20''' provided to extend in the circumferential direction along the peripheral surface of the head including the forehead of the user, and in the present embodiment a pair of the pressing portions 20''' is provided. The pair of the pressing portions 20''' has a length that allows to extend half around the inner periphery of the attachment member 10''' in the circumferential direction, is formed into a barrel shape extending in the circumferential direction of the attachment member body 11''', and is provided at a distance in a direction (that is, a vertical direction) perpendicular to the circumferential direction and parallel to the peripheral surface of the head including the forehead of a user. In the pair of the pressing portions 20''', a columnar elastic member formed of resin such as urethane is covered with a woven rubber piece, and the woven rubber piece is sewn on the inside of the attachment member body 11''' to be attached to the inside of the attachment member body 11'''. In the posture correcting tool 1''' according to the fourth embodiment, although the pair of the pressing portions 20''' is provided, the present invention is not limited thereto, and one or three or more pressing portions may be provided.

The posture correcting tool 1''' according to the fourth embodiment is attached to the head including the forehead of a user, whereby the forehead of the user is mainly pressed on line by the pair of the pressing portions 20'''. In the posture correcting tool 1''' according to the fourth embodiment, the pressing portion 20''' may be removable from a holding portion of the attachment member 10''' as in the first to third embodiments, and the number and position thereof may be suitably adjustable.

As described above, according to the posture correcting tools 1, 1', 1'', and 1''' according to the first to fourth embodiments, it is possible to correct forward tilting of the head and distortion that cannot be corrected by the posture correcting tools disclosed in the Patent Literatures 1 and 2, and a good posture can be maintained. Since the posture correcting tools 1, 1', 1'', and 1''' according to the first to fourth embodiments presses the peripheral surface of the head including the forehead of a user to correct the posture of the user, the structure is simple, and the posture correcting tools can be used by such a simple operation as to attach the posture correcting tools to the head of the user.

The posture correcting tools 1, 1', 1'', and 1''' according to the first to fourth embodiments return the head of a user to a proper position and can correct the posture into a suitable posture. Accordingly, the posture correcting tool can be used not only in treatment of a so-called hunchback and the like but also in treatment of symptoms such as whiplash and cervical disc herniation and form correction in sports such as golf. Namely, according to the posture correcting tools 1, 1', 1'', and 1''' according to the first to fourth embodiments, the head of a user is returned to a proper position with ease, so that neck pain is reduced, and, at the same time, the weight applied to the head can be reduced. Therefore, instead of conventionally used medical instruments such as a collar (neck corset), the posture correcting tools can be used as new medical instruments having excellent appearance (design) for symptoms such as whiplash and cervical disc herniation. Particularly, in the posture correcting tool 1'' according to the third embodiment, the pressing portion 20'' is provided inside the hook-and-loop fastener 12', whereby the pressing portion 20'' can be made invisible from outside, and therefore, an excellent design can be obtained. Further, since the posture correcting tool is formed into a hat shape, the posture correcting tool can be actively used in the form correction in sports such as golf. When the posture correcting tools 1, 1', 1'', and 1''' according to the first to fourth embodiments are used in the form correction in sports such as golf, the center of gravity of a user is corrected into a proper position, and, at the same time, look up and form can be properly corrected, whereby swing can be performed without putting muscle on the body. Therefore, the load on neck and shoulders can be reduced.

The posture correcting tool according to the present invention is not limited to the above embodiments, and various alterations can be made within a range without departing the technical spirit of the invention. For example, the attachment members 10, 10', and 10''' of the posture correcting tools 1, 1', and 1''' according to the first, second, and fourth embodiments are formed into a ring shape, and the attachment member 10'' of the posture correcting tool 1'' according to the third embodiment is formed into a hat shape; however, the present invention is not limited thereto, and the attachment member may be formed into various shapes such as a helmet shape as long as it can be attached to the head of a user. In this case, various constitutions can be adopted. For example, an actuator is provided in the attachment member, and a pressing portion is pressed against the peripheral surface of the head including the forehead of a user by the actuator. Meanwhile, a pressing portion is formed into an expandable air bag shape, and air is supplied to expand the pressing portion, and, thus, to press the pressing portion against the peripheral surface of the head including the forehead of a user.

In the pressing portions 20, 20', 20", and 20''' of the posture correcting tools 1, 1', 1'', and 1''' according to the first to fourth embodiments, at least one pressing portion may be provided. In the posture correcting tools 1, 1', 1'', and 1''' according to the first to fourth embodiments, although the pressing portions 20, 20', 20'', and 20''' are formed into a columnar shape, a prismatic shape, or a barrel shape, the present invention is not limited thereto, and the pressing portions may be formed into any shape.

Although the attachment members 10, 10', and 10''' of the posture correcting tools 1, 1', and 1''' according to the first, second, and fourth embodiments are formed into a ring shape by the hook-and-loop fasteners 12, 12', and 12''', the present invention is not limited thereto, and the hook-and-loop fasteners may not be provided by forming the belt-like attachment member body into a ring shape.

### Reference Signs List

1, 1', 1'', 1''' Posture correcting tool, 2 User, 3 Forehead, 10, 10', 10", 10''' Attachment member, 20, 20', 20", 20''' Pressing portion

## Claims

1. A posture correcting tool comprising:
an attachment member attachable to a head of a user;
and
at least one pressing portion which is provided in the attachment member, protrudes from the attachment member toward the head of the user, and can press at least a portion of a peripheral surface of the head including a forehead of the user.

2. The posture correcting tool according to claim 1, wherein at least two pressing portions are provided along the peripheral surface of the head including the forehead of the user at a distance in the circumferential direction.

3. The posture correcting tool according to claim 1, wherein the pressing portion is provided to extend in the circumferential direction along the peripheral surface of the head including the forehead of the user.

4. The posture correcting tool according to claim 3, wherein at least two pressing portions are provided at a distance in a direction that is perpendicular to the circumferential direction and parallel to the peripheral surface of the head including the forehead of the user.

5. The posture correcting tool according to claim 2 or 4, wherein the pressing portion is configured so that an interval between the pressing portion and another adjacent pressing portion can be adjusted.

6. The posture correcting tool according to any one of claims 1 to 5, wherein the pressing portion is configured so that a position with respect to the peripheral surface of the head including the forehead of the user can be adjusted.

7. The posture correcting tool according to any one of claims 1 to 6, wherein the attachment member is formed into a ring shape attachable to the peripheral surface of the head including the forehead of the user.

8. The posture correcting tool according to any one of claims 1 to 6, wherein the attachment member is formed into a hat shape attachable to the head of the user.

9. The posture correcting tool according to any one of claims 1 to 8, wherein at least a portion of the attachment member is formed of an elastic material, and the pressing portion is configured to press at least a portion of the peripheral surface of the head including the forehead of the user by contractive force of the attachment member.
